# EUROPEAN PATENT SPECIFICATION

(11) **EP 1 976 625 B1**
(45) Date of publication and mention of the grant of the patent: **03.06.2009**
(21) Application number: 06841526.4
(22) Date of filing: 20.12.2006
(51) Int. Cl.: B01J 19/00, C07D 301/08

(54) **Process and apparatus for concentrating and purifying ethylene oxide**
Verfahren und Vorrichtung zur Konzentration und Reinigung von Ethylenoxid
Procédé et appareil pour la concentration et purification d'oxyde d'éthylène

(30) Priority: 22.12.2005 EP 05257995
(43) Date of publication of application: 08.10.2008
(73) Proprietor: Shell Internationale Research Maatschappij B.V., 2596 HR Den Haag (NL)
(72) Inventor: REKERS, Dominicus Maria, NL-1031 CM Amsterdam (NL); SLAPAK, Mathias Jozef Paul, NL-1031 CM Amsterdam (NL)
(74) Representative: Zeestraten, Albertus W. J.
(86) International application number: PCT/EP2006/070030
(87) International publication number: WO 2007/071739

(56) References cited:
- WO-A-99/64147
- WO-A-03/049835
- WO-A-20/04016347
- WO-A-20/04103539
- WO-A-20/06020709
- WO-A-20/06053345
- WO-A-20/06055609
- WO-A-20/06127889
- US-A1- 2004 220 434
- US-A1- 2006 036 106

## Description

### Field of the Invention

The present invention relates to improvements in process operations involving hydrocarbons. The process improvements envisaged find application in the production of etylene oxide from alkylene and oxygen and in its optional further conversion.

### Background of the Invention

When operating on a commercial scale, process operations have to meet a number of important design criteria. In the modern day environment, process design has to take account of environmental legislation and keep to health and safety standards. Processes that utilise or produce dangerous chemicals pose particular problems and often, in order to minimise risks of explosion or reaction runaway, such process operations have to be run at conditions that are not optimal; this increases the running costs of a plant (the operational expenditure or OPEX). Such processes may also have to utilise more equipment than is necessary just to perform the process; this leads to an increase in building costs (the capital expenditure or CAPEX).

There is an on-going need to provide process operations that can reduce CAPEX and OPEX costs and particularly without increasing the risk of damage to the plant and danger to the public and/or to the process plant workers.

### Summary of the Invention

The present invention provides for the utilisation of microchannel apparatus in process operations. Such apparatus have previously been proposed for use in certain specific fields of application but have not previously been proposed to provide the combination of reduced CAPEX and/or OPEX with maintained or reduced plant safety risks.

WO 99/64147 discloses the use of microchannel devices in various unit operations such as absorption, adsorption, desorption, distillation and combinations thereof. WO 03/49835 discloses a microchannel device used in the separation of fluids.

The invention provides a process for the concentration or purification of ethylene oxide, which comprises
a) absorbing ethylene oxide from a first gaseous stream which comprises ethylene oxide with a suitable absorbent,
b) desorption of the ethylene oxide in a distillation unit to form a second gaseous stream containing ethylene oxide, and
c) recovering ethylene oxide,
wherein the second gaseous stream of step b) is condensed in one or more process microchannels in a microchannel apparatus, and the microchannel apparatus is positioned inside the shell of a distillation column at a point above the uppermost distillation tray or packing material.

The invention further provides an apparatus for the concentration or purification of ethylene oxide from a mixture of ethylene oxide and water, which comprises a distillation column and a microchannel apparatus, wherein the microchannel apparatus is positioned inside the shell of the distillation column at a point above the uppermost distillation tray or packing material.

### Brief Description of the Drawings

FIG. 1 shows a schematic drawing of a microchannel reactor and its main constituents.
FIG. 2 shows a schematic drawing of a typical example of a repeating unit which comprises process microchannels and heat exchange channels and its operation when in use in the practice of the invention. A microchannel apparatus or reactor utilised in this invention may comprise a plurality of such repeating units.
FIG. 3 shows a schematic drawing of an example of a process for the purification of ethylene oxide according to the invention.

### Detailed Description of the Invention

The present invention provides processes that utilise microchannel apparatus for physical operations. Hereinafter a discussion of such apparatus is given.

Microchannel reactors suitable for use in this invention and their operation have been described in WO-A-2004/099113, WO-A-01/12312 , WO-01/54812, US-A-6440895, US-A-6284217, US-A-6451864, US-A-6491880, US-A-6666909, US-A-6811829, US-A-6851171, US-A-6494614, US-A-6228434 and US-A-6192596. Methods by which the microchannel reactor may be manufactured and operated, as described in these references, may generally be applicable in the practice of the present invention.

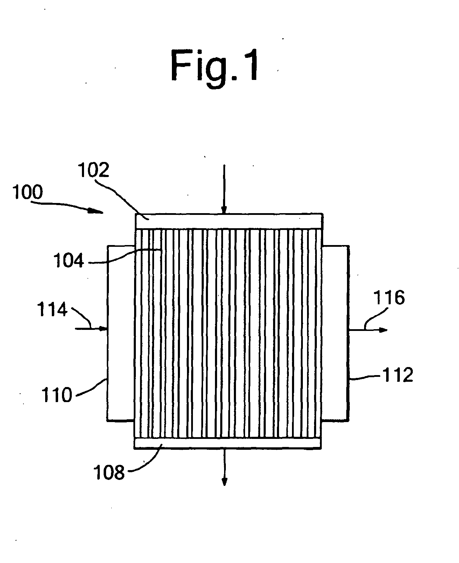

With reference to FIG. 1, microchannel reactor 100 may be comprised of a header 102, a plurality of process microchannels 104, and a footer 108. The header 102 provides a passageway for fluid to flow into the process microchannels 104. The footer 108 provides a passageway for fluid to flow from the process microchannels 104.

The number of process microchannels contained in a microchannel reactor may be very large. For example, the number may be up to 10⁵, or even up to 10⁶ or up to 2 x 10⁶. Normally, the number of process microchannels may be at least 10 or at least 100, or even at least 1000.

The process microchannels are typically arranged parallel, for example they may form an array of planar microchannels. Each of the process microchannels may have at least one internal dimension of height or width of up to 15 mm, for example from 0.05 to 10 mm, in particular from 0.1 to 5 mm, more in particular from 0.5 to 2 mm. The other internal dimension of height or width may be, for example, from 0.1 to 100 cm, in particular from 0.2 to 75 cm, more in particular from 0.3 to 50 cm. The length of each of the process microchannels may be, for example, from 1 to 500 cm, in particular from 2 to 300 cm, more in particular from 3 to 200 cm, or from 5 to 100 cm.

The microchannel reactor 100 additionally comprises heat exchange channels (not shown in FIG. 1) which are in heat exchange contact with the process microchannels 104. The heat exchange channels may be microchannels. The microchannel reactor is adapted such that heat exchange fluid can flow from heat exchange header 110 through the heat exchange channels to heat exchange footer 112. The heat exchange channels may be aligned to provide a flow in a co-current, counter-current or, in some aspects, preferably cross-current direction, relative to a flow in the process microchannels 104. The cross-current direction is as indicated by arrows 114 and 116.

Each of the heat exchange channels may have at least one internal dimension of height or width of up to 15 mm, for example from 0.05 to 10 mm, in particular from 0.1 to 5 mm, more in particular from 0.5 to 2 mm. The other internal dimension of height or width may be, for example, from 0.1 to 100 cm, in particular from 0.2 to 75 cm, more in particular from 0.3 to 50 cm. The length of each of the heat exchange channels may be, for example, from 1 to 500 cm, in particular from 2 to 300 cm, more in particular from 3 to 200 cm, or from 5 to 100 cm.

The separation between each process microchannel 104 and the next adjacent heat exchange channel may be in the range of from 0.05 mm to 5 mm, in particular from 0.2 to 2 mm.

In some embodiments of this invention, there is provided for first heat exchange channels and second heat exchange channels, or first heat exchange channels, second heat exchange channels and third heat exchange channels, or even up to fifth heat exchange channels, or even further heat exchange channels. Thus, in such cases, there is a plurality of sets of heat exchange channels, and accordingly there may be a plurality of heat exchange headers 110 and heat exchange footers 112, whereby each set of heat exchange channels may be adapted to receive heat exchange fluid from a heat exchange header 110 and to deliver heat exchange fluid into a heat exchange footer 112.

The header 102, footer 108, heat exchange header 110, heat exchange footer 112, process microchannels 104 and heat exchange channels may independently be made of any construction material which provides sufficient strength, optionally dimensional stability, and heat transfer characteristics to permit operation of the processes in accordance with this invention. Suitable construction materials include, for example, steel (for example stainless steel and carbon steel), monel, titanium, copper, glass and polymer compositions. The kind of heat exchange fluid is not material to the present invention and the heat exchange fluid may be selected from a large variety. Suitable heat exchange fluids include steam, water, air and oils. In embodiments of the invention which include a plurality of sets of heat exchange channels, such sets of heat exchange channels may operate with different heat exchange fluids or with heat exchange fluids having different temperatures.

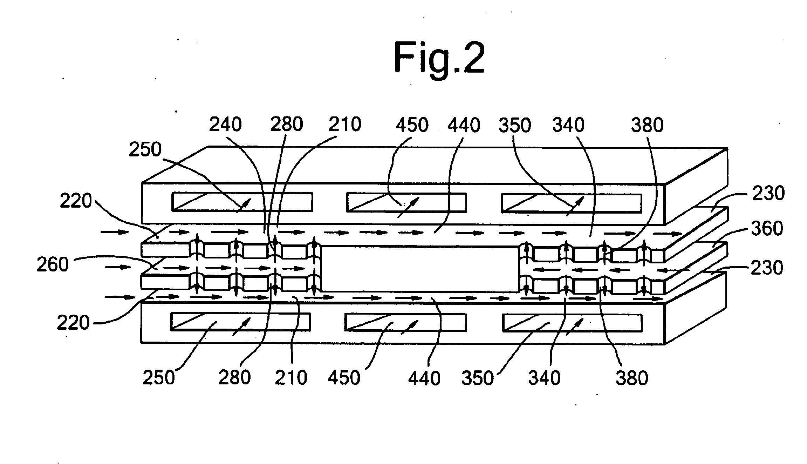

A microchannel reactor of use in the invention may comprise a plurality of repeating units each comprising one or more process microchannels and one or more heat exchange channels. Reference is now made to FIG. 2, which shows a typical repeating unit and its operation.

Process microchannels 210 have an upstream end 220 and a downstream end 230 and may comprise of a first section 240. First section 240 may be in heat exchange contact with first heat exchange channel 250, allowing heat exchange between first section 240 of process microchannel 210 and first heat exchange channel 250. The repeating unit may comprise first feed channel 260 which leads into first section 240 through one or more first orifices 280. Typically one or more first orifices 280 may be positioned downstream relative to another first orifice 280. During operation, feed may enter into first section 240 of process microchannel 210 through an opening in upstream end 220 and/or through first feed channel 260 and one or more first orifices 280.

Process microchannels 210 may comprise a second section 340. Second section 340 is positioned down stream of first section 240. Second section 340 may be in heat exchange contact with second heat exchange channel 350, allowing heat exchange between second section 340 of process microchannel 210 and second heat exchange channel 350. In some embodiments second section 340 is adapted to quench product obtained in and received from first section 240 by heat exchange with a heat exchange fluid in second heat exchange channel 350. Quenching if required may be achieved in stages by the presence of a plurality of second heat exchange channels 350, for example two or three or four. Such a plurality of second heat exchange channels 350 may be adapted to contain heat exchange fluids having different temperatures, in particular such that in downstream direction of second section 340 heat exchange takes place with a second heat exchange channel 350 containing a heat exchange fluid having a lower temperature. The repeating unit may comprise second feed channel 360 which leads into second section 340 through one or more second orifices 380. During operation, feed may enter into second section 340 from upstream in process microchannel 210 and through second feed channel 360 and one or more second orifices 380.

The first and second feed channels 260 or 360 in combination with first and second orifices 280 or 380 whereby one or more first or second orifices 280 or 380 are positioned downstream to another first or second orifice 280 or 380, respectively, allow for replenishment of a reactant. Replenishment of a reactant can be utilised in some embodiments of this invention.

Process microchannels 210 may comprise an intermediate section 440, which is positioned downstream of first section 240 and upstream of second section 340. Intermediate section 440 may be in heat exchange contact with third heat exchange channel 450, allowing heat exchange between intermediate section 440 of the process microchannel 210 and third heat exchange channel 450.

In some embodiments, process microchannel 210 may comprise a third section (not drawn) downstream of second section 340, and optionally a second intermediate section (not drawn) downstream of second section 340 and upstream of the third section. The third section may be in heat exchange contact with a fourth heat exchange channel (not drawn), allowing heat exchange between the third section of the process microchannel 210 and fourth heat exchange channel. The second intermediate section may be in heat exchange contact with a fifth heat exchange channel (not drawn), allowing heat exchange between the second intermediate section of the process microchannel 210 and fifth heat exchange channel. The repeating unit may comprise a third feed channel (not drawn) which ends into the third section through one or more third orifices (not drawn). Typically one or more third orifices may be positioned downstream relative to another third orifice. During operation, feed may enter into the third section from upstream in process microchannel 210 and through the third feed channel and the one or more third orifices.

Each of the feed channels may be a microchannel. They may have at least one internal dimension of height or width of up to 15 mm, for example from 0.05 to 10 mm, in particular from 0.1 to 5 mm, more in particular from 0.5 to 2 mm. The other internal dimension of height or width may be, for example, from 0.1 to 100 cm, in particular from 0.2 to 75 cm, more in particular from 0.3 to 50 cm. The length of each of the feed channels may be, for example, from 1 to 250 cm, in particular from 2 to 150 cm, more in particular from 3 to 100 cm, or from 5 to 50 cm.

The length of each of the sections of the process microchannels may be selected independently of each other, in accordance with, for example, the heat exchange capacity needed. The lengths of the sections may independently be at least 1 cm, or at least 2 cm, or at least 5 cm. The lengths of the sections may independently be at most 250 cm, or at most 150 cm, or at most 100 cm, or at most 50 cm. Other dimensions of the sections are defined by the corresponding dimensions of process microchannel 210.

The microchannel reactor of this invention may be manufactured using known techniques, for example conventional machining, laser cutting, molding, stamping and etching and combinations thereof. The microchannel reactor of this invention may be manufactured by forming sheets with features removed which allow passages. A stack of such sheets may be assembled to form an integrated device, by using known techniques, for example diffusion bonding, laser welding, cold welding, diffusion brazing, and combinations thereof. The microchannel reactor of this invention comprises appropriate headers, footers, valves, conduit lines, and other features to control input of reactants, output of product, and flow of heat exchange fluids. These are not shown in the drawings, but they can be readily provided by those skilled in the art. Also, there may be further heat exchange equipment (net shown in the drawings) for temperature control of feed, in particular for heating feed or feed components, before it enters the process microchannels, or for temperatures control of product, in particular for cooling product, after it has left the process microchannels. Such further heat exchange equipment may be integral with the microchannel reactor, but more typically it will be separate equipment. These are not shown in the drawings, but they can be readily provided by those skilled in the art.

The present invention finds application in a process for the manufacture of ethylene oxide, by the direct epoxidation of alkylene using oxygen or air, see Kirk-Othmer Encyclopedia of Chemical Technology, 3rd edition, Volume 9, 1980, pages 445 to 447. In the air-based process, air or air enriched with oxygen is employed as a source of the oxidizing agent while in the oxygen-based processes, high purity (at least 95 mole%) oxygen is employed as the source of the oxidising agent. Currently most epoxidation plants are oxygen-based. The epoxidation process may be carried out using reaction temperatures selected from a wide range. Preferably the reaction temperature within the epoxidation reactor is in the range of from 150 °C to 340 °C, more preferably in the range of from 180 to 325 °C. The reaction is preferably carried out at a pressure of in the range of from 1000 to 3500 kPa.

A process operation that presents an explosion risk, particularly in an ethylene oxide plant, is the handling of ethylene oxide itself. Ethylene oxide (EO) is an unstable and very reactive component. In equipment that contains EO vapour, several reactions can occur which are exothermic. Where the heat of reaction is not removed fast enough, the temperature in the equipment can increase rapidly and, if unchecked, can lead to explosive decomposition reactions of the EO vapour. Where additional substances are present then explosive reactions can occur at lower temperatures than for pure ethylene oxide. Even EO liquid under certain circumstances can be dangerous.

In a commercial ethylene oxide production plant, the sections for which this is of most concern are the EO concentrator and the EO purification sections. The EO concentrator is often also called the EO stripper. Both EO concentrator and the EO purification sections utilise a distillation column, to separate EO from water, which may be equipped with a condenser and an EO condensate collection vessel. In the latter, stagnant, liquid EO exists possibly in conjunction with water. In order to reduce the chance of explosive decomposition reactions, whether in a distillation column or in an EO condensate collection vessel, the top section plus overhead system of an EO concentrator column and of an EO purification column are conventionally operated under nitrogen pressure, which increases the operating pressure by at least a factor of 1.7. The pressure can also be generated by use of a gas other than nitrogen, which may be selected from one or more of carbon dioxide, methane and a process gas such as a light ends gas. Usually, however, nitrogen is used. It would be most desirable to be able to operate these columns without the need for pressurisation in the top section, yet still have a low explosion risk.

The use of microchannel apparatus has the advantage of being able to cool an ethylene oxide-containing mixture very efficiently, thus minimising the likelihood of explosive decomposition reactions. Since there exist a large number of process microchannels within a microchannel apparatus, and because of the dimensions of the process microchannels, the EO-containing feed is split up into multiple small volumes. A heat transfer medium is run through heat exchange channels of the apparatus to ensure a rapid heat flux from EO to heat transfer medium. These features ensure a high efficiency of heat transfer and minimise the volume of gas that can be in the explosive region. Furthermore the nature of the process microchannels means that the apparatus can act as a flame arrester and provide an intrinsically safe condensation system for EO-containing gases. In this aspect of the present invention, it is thus also preferred to use a microchannel apparatus having one or more, and preferably all, process microchannels having an internal dimension of height and/or width of at most 5 mm, most preferably at most 2 mm, and especially at most 1.5 mm. Said internal dimension is preferably at least 0.1 mm, most preferably at least 0.5 mm, and especially at least 0.5 mm.

The use of microchannel apparatus can provide a much larger heat transfer than conventional shell and tube heat exchangers, and is a much smaller item of equipment. Thus a CAPEX improvement is given by the combination of smaller condensation equipment as well as by the removal of reflux drums. The apparatus also has the potential to reduce the need for excess pressurisation of the upper sections of these columns. The OPEX may also be improved by any reduced pressurisation in the top section of these EO distillation columns. Where the pressure can be reduced then there is additional significant advantage, particularly in an EO stripper or concentrator, in that a lower temperature steam can be used to heat the column and the amount of glycol byproduct can be reduced. Furthermore the microchannels apparatus acts as a flame arrester and provides an intrinsically safe EO condensation system.

Suitable absorbents that can absorb ethylene oxide are documented in literature and include water (see Research Disclosure No. 465117, idem); ethylene carbonate (see US-A-4,221,727 and EP-A-776890); propylene carbonate (EP-A-705826); aqueous ethylene glycol solutions having a glycol content up to 40% and antifoam additive content of up to 500 ppm (US-A-4,875,909 and GB-A-1435848); methanol (US-A-3,948,621); organic liquid solvents (US-A-4,249,917); and liquid hydrocarbons such as methane, ethane and/or ethylene in liquid form (US-A-3,644,432). However, most commonly water or an aqueous solution is utilised and is preferred in the process of the present invention.

In step a) absorption of ethylene oxide with water or an aqueous solution creates an aqueous solution of ethylene oxide. In step b) the ethylene oxide is then desorbed by dewatering in a distillation unit.

A distillation unit may comprise one or more distillation columns. Most suitably a maximum of five distillation columns are utilised in series within a unit forming a 'distillation train'. Preferably a distillation unit comprises from one to three distillation columns, most preferably only one distillation column. Where a single column is utilised in step b) the second gaseous stream is obtained in the upper section of the column, and an aqueous product is given in the bottom section. In a distillation train of columns equivalent product streams are obtained at appropriate points, as would be well known to the skilled person in the art.

The term 'dewatering' herein should be understood to mean the removal of water.

In a preferred embodiment, step a) is the absorption of ethylene oxide in an aqueous solution to produce a stream in which water is enriched with EO and step b) is performed in an EO stripper or concentrator, which is a single distillation column. While the EO is stripped or concentrated from the aqueous feed stream, the product drawn off from the upper section, and preferably drawn off at the very top, of the distillation column is still a mixture of ethylene oxide and water. This gaseous mixture product is condensed in a microchannel apparatus and the resulting EO-containing stream can be utilised for the production of other chemicals such as 1,2-diols, 1,2-diol ethers, 1,2-carbonates or alkanol amines by processes known in the art, or it can be further purified to yield a high purity EO. A portion of said resulting EO-containing stream may be recycled to the EO concentrator column, and a bleed of gases, such as methane, CO₂ and ethylene, can be drawn off by procedures well known to those skilled in this art. In this embodiment, the first gaseous stream is an EO-containing product stream of a reactor in which ethylene and oxygen are reacted to form ethylene oxide. Preferably the first gaseous stream comprises EO in the range of from 2 to 50 % by weight, more preferably from 2 to 10 % by weight, and especially from 4 to 6 % by weight. The aqueous solution of step a) primarily comprises water in an amount from 50 to 100 % by weight. It is possible that in the range of from 0.1 to 20, e.g. 2 to 10, % by weight of said aqueous solution is a glycol, mostly being mono-ethylene glycol. In such solutions an anti-foam additive is not required but may be utilised if desired.

In an alternative preferred embodiment step b) may be performed several stages downstream of step a) and in the EO purification section of an EO production process, in which case the second gaseous stream will contain predominantly EO, with trace amounts of impurities. Thus less than 10,000 ppm, ie for example from 1 ppm to 10,000 ppm, of other compounds may also be present. Such compounds may comprise for example water, carbon dioxide, argon, nitrogen, oxygen, aldehydes, such as acetaldehyde, and formaldehyde, and, as above, other hydrocarbons, alcohols, acids, acetals, cyclic acetals, ethers, cyclic ethers, and esters. In this embodiment, the second gaseous stream is purified ethylene oxide which may be drawn off at any point in the upper section of the distillation column, thus it may be drawn off directly in gaseous form via a top draw-off, above the upper plate or internal packing, or via a gaseous or liquid side draw-off below the upper tray or upper level of packing.

In both embodiments, it is preferred that the second gaseous stream comprises 50 % by weight or more of EO.

In a further aspect of the present invention, apparatus is provided for the concentration or purification of ethylene oxide from a mixture of ethylene oxide and water, which is a distillation column connected to a microchannel apparatus. Advantageously, the microchannel apparatus is positioned inside the shell of the distillation column at a point above the uppermost distillation tray or packing material.

By incorporating microchannel apparatus inside the distillation column it is possible to provide integral reflux within the column which significantly improves process safety. In a commercial EO production plant, such use, in accordance with present invention, of an integrated microchannel apparatus to cool EO gases within a distillation column allows cooling, by condensation, to occur without the need of an external condensate collection vessel. The presence of stagnant EO is therefore avoided and the likelihood of explosion is minimised.

The operation of the process of the present invention can be described, for example, with reference to FIG. 1 herewith.

The gaseous mixture comprising ethylene oxide, which preferably is a gaseous stream forming the top stream of an EO stripper or of an EO purification column, enters header 102 and is split into multiple portions to progress through the reactor via a plurality of process microchannels 104. Coolant is fed into the apparatus via heat exchange header 110 and flows through the apparatus cross-currently (as shown in FIG. 1) or co- or counter- currently, via heat exchange channels to the footer 112.

The microchannel apparatus is sited within a distillation column, and is most suitably sited in the centre of the column and may extend across the full diameter of the column, being affixed directly to the column walls, or across the diameter only in part. In the latter case the apparatus may be placed on beams extending from the inner column walls or may be suspended by arms extending from the inner column walls, provided that the beams or arms do not restrict the gas and liquid flow in the column. The height of the microchannel apparatus suitably also is such as to not interfere with the normal operation of the distillation column; and the length of the process microchannels most suitably is in the range of from 5 to 100 cm.

In all cases it is important that gas flow in the distillation column can circulate to the top of the column either around the outside of the microchannel apparatus or through channels or holes in the apparatus. The microchannel apparatus is so sited that the second gaseous stream enters the apparatus via a header at the top of the apparatus as in FIG. 1. The gaseous stream condenses within the process microchannels and liquid ethylene oxide runs through the process microchannels and collects in an exit header, or other collection unit, at or under the bottom of the apparatus, and can be withdrawn from the column. The condensation causes the gaseous stream automatically to be drawn into the process microchannels. Coolants can be routed into the apparatus and may for example be water or other coolant material that takes up heat created by the condensation.

The general process conditions that may apply for the use of microchannel apparatus in an EO condensation process of the invention are suitably a temperature in the range of from ambient (20 °C) to 100 °C, for example 30 to 50 °C, and a pressure in the range of from 100 to 1,000 kPa, for example 200 to 400 kPa.

The present invention will now be illustrated by the following Example.

### Example

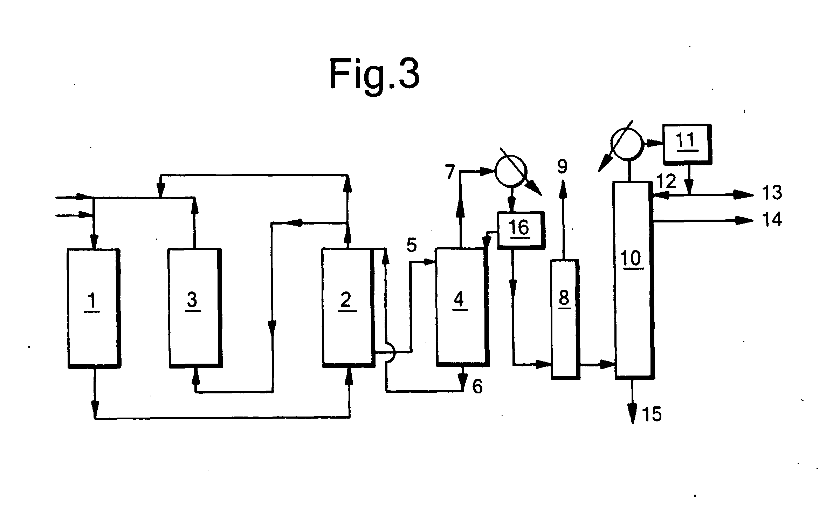

In a 400,000 mt/a ethylene oxide plant the stream of recycle gas to the reactor system is 600 mt/h. This flow mainly consists of methane, ethylene, oxygen, argon, carbon dioxide and nitrogen. The temperature at the reactor inlet is 140°C and the pressure is 2000 kPa gauge. In FIG. 3, over the catalyst inside the reactor 1, ethylene oxide and carbon dioxide are produced. EO is scrubbed in the EO absorber 2 and part of the recycle gas is scrubbed of CO₂ in the CO₂ absorber 3. The absorbent used for EO scrubbing is typically water with a small concentration of monoethylene glycol (2-10 weight%). Water saturated with ethylene oxide via line 5 from the bottom of the EO absorber 2 is fed to the top of EO stripper 4. The bottoms flow, line 6, of EO stripper 4 is virtually free of EO and recycled back to the top of EO absorber 3. An ethylene oxide-water mixture (typically containing 50 to almost 100 weight% ethylene oxide) is boiled over the top of the EO stripper as a vapour flow, shown by line 7, and is condensed into vessel 16. Optionally part of the condensed vapour can be refluxed to increase the EO concentration in the top of the EO stripper. Gases like methane, CO₂ and ethylene are removed, via line 9, from the condensed water/ethylene oxide mixture in a light ends column 8. For pure EO applications the EO is dehydrated and purified in EO purification column 10. Water or a mixture of water and EO leaves the bottom of this column via line 15. The top vapour is condensed and largely refluxed and re-enters the column via line 12 from condensate collection/reflux vessel 11. Typically a small EO flow is fed from the EO reflux drum 11 to the glycol section as a bleed for light components (see line 13). The pure EO product flow (line 14) is taken from the top section of this column, in this example a few trays below the reflux tray.

In this example the top of the EO stripper 4, the top stripper condensers, EO/water line 15, the light ends column 8, the top section of the EO purification column 10, and the EO reflux drum 11 contain a high concentration of EO. To limit explosion hazard both EO condensers are microchannel apparatus such as described with respect to FIG. 1 and FIG. 2. They act as condensers according to the present invention. In the apparatus the total EO volume is divided into a large amount of small volumes inside the microchannels. In addition to that, heat transfer is drastically increased, thus minimizing the risk of runaway reactions eventually leading to explosions. In this example the microchannel condensers can optionally be integrated inside the EO stripper 4 and EO purification column 10 as a so-called cold finger enabling internal reflux. Thus a large volume of EO in a reflux vessel is avoided and explosion risk is even further reduced.

## Claims

1. A process for the concentration or purification of ethylene oxide, which comprises
a) absorbing ethylene oxide from a first gaseous stream which comprises ethylene oxide with a suitable absorbent,
b) desorption of the ethylene oxide in a distillation unit to form a second gaseous stream containing ethylene oxide, and
c) recovering ethylene oxide,
wherein the second gaseous stream of step b) is condensed in one or more process microchannels in a microchannel apparatus, and the microchannel apparatus is positioned inside the shell of a distillation column at a point above the uppermost distillation tray or packing material.

2. A process as claimed in claim 1, wherein in step b) the second gaseous stream comprises 50 % by weight or more of ethylene oxide.

3. A process as claimed in claim 1 or claim 2, wherein in step b) the second gaseous stream comprises ethylene oxide and less than 10,000 ppm by weight of other compounds, and purified ethylene oxide is recovered separately from the upper section of a single distillation column via a top or side draw-off.

4. A process as claimed in any one of claims 1 to 3 wherein the microchannel apparatus comprises one or more process microchannels having an internal height and/or width in the range of from 0.5 to 1.5 mm.

5. Apparatus for the concentration or purification of ethylene oxide from a mixture of ethylene oxide and water, which comprises a distillation column and a microchannel apparatus, wherein the microchannel apparatus is positioned inside the shell of the distillation column at a point above the uppermost distillation tray or packing material.

6. Apparatus as claimed in claim 5, wherein the microchannel apparatus comprises one or more process microchannels having an internal height and/or width in the range of from 0.5 to 1.5 mm, and a length in the range of from 5 to 100 cm.

## Patentansprüche

1. Verfahren zur Konzentrierung oder Reinigung von Ethylenoxid, welches
a) das Absorbieren von Ethylenoxid aus einem ersten gasförmigen Strom, welcher Ethylenoxid umfasst, mit einem geeigneten Absorptionsmittel,
b) die Desorption des Ethylenoxids in einer Destillationseinheit, um einen zweiten gasförmigen Strom auszubilden, welcher Ethylenoxid enthält, und
c) das Gewinnen von Ethylenoxid,
wobei der zweite gasförmige Strom aus Schritt b) in einem oder mehreren Verfahrensmikrokanälen in einer Mikrokanalapparatur kondensiert wird, und die Mikrokanalapparatur im Inneren des Deatillationskolonnengehäuses an einem Punkt oberhalb des höchsten Destillationsbodens oder des Packungsmaterials angeordnet ist, umfasst.

2. Verfahren nach Anspruch 1, wobei im Schritt b) der zweite gasförmige Strom 50 Gew.-% oder mehr an Ethylenoxid umfasst.

3. Verfahren nach Anspruch 1 oder Anspruch 2, wobei im Schritt b) der zweite gasförmige Strom Ethylenoxid und weniger als 10000 ppm, bezogen auf das Gewicht, an anderen Verbindungen umfasst, und das gereinigte Ethylenoxid getrennt aus dem oberen Abschnitt einer einzelnen Destillationskolonne über eine Kopf- oder eine Seitenentnahme gewonnen wird.

4. Verfahren nach einem der Ansprüche 1 bis 3, wobei die Mikrokanalapparatur einen oder mehrere Verfahrensmikrokanäle mit einer Innenhöhe und/oder -weite im Bereich von 0,5 bis 1,5 mm umfasst.

5. Apparatur zur Konzentrierung oder Reinigung von Ethylenoxid aus einem Gemisch von Ethylenoxid und Wasser, welche eine Destillationskolonne und eine Mikrokanalapparatur umfasst, wobei die Mikrokanalapparatur im Inneren des Destillationskolonnengehäuses an einem Punkt oberhalb des höchsten Destillationsbodens oder des Packungsmaterials angeordnet ist.

6. Apparatur nach Anspruch 5, wobei die Mikrokanalapparatur einen oder mehrer Verfahrensmikrokanäle mit einer Innenhöhe und/oder -weite im Bereich von 0,5 bis 1,5 mm, und einer Länge im Bereich von 5 bis 100 cm umfasst.

## Revendications

1. Procédé pour la concentration ou la purification d'oxyde d'éthylène, comprenant les étapes suivantes:
a) absorber de l'oxyde d'éthylène à partir d'un premier courant de gaz qui contient de l'oxyde d'éthylène en utilisant un absorbant approprié;
b) désorber l'oxyde d'éthylène dans une unité de distillation pour former un deuxième courant de gaz contenant de l'oxyde d'éthylène; et
c) récupérer l'oxyde d'éthylène,
dans lequel le deuxième courant de gaz de l'étape b) est condensé dans un micro-canal ou plusieurs micro-canaux de traitement dans un dispositif à micro-canaux, et le dispositif à micro-canaux est positionné à l'intérieur de l'enveloppe d'une colonne de distillation en un point qui est situé au-dessus du plateau de distillation le plus élevé ou de la matière de garniture la plus haute.

2. Procédé selon la revendication 1, dans lequel, à l'étape b), le deuxième courant de gaz contient 50 % en poids ou plus d'oxyde d'éthylène.

3. Procédé selon la revendication 1 ou la revendication 2, dans lequel, à l'étape b), le deuxième courant de gaz contient de l'oxyde d'éthylène et moins de 10 000 ppm en poids d'autres composés, et dans lequel l'oxyde d'éthylène purifié est récupéré séparément à partir de la section supérieure d'une seule colonne de distillation par l'intermédiaire d'un soutirage supérieur ou latéral.

4. Procédé selon l'une quelconque des revendications 1 à 3, dans lequel le dispositif à micro-canaux comprend un micro-canal ou plusieurs micro-canaux de traitement dont la hauteur intérieure et/ou la largeur intérieure est (sont) comprise(s) dans la gamme de 0,5 mm à 1,5 mm.

5. Appareil pour la concentration ou la purification d'oxyde d'éthylène à partir d'un mélange d'oxyde d'éthylène et d'eau, comprenant une colonne de distillation et un dispositif à micro-canaux, dans lequel le dispositif à micro-canaux est positionné à l'intérieur de l'enveloppe de la colonne de distillation en un point qui est situé au-dessus du plateau de distillation le plus élevé ou de la matière de garniture la plus haute.

6. Appareil selon la revendication 5, dans lequel le dispositif à micro-canaux comprend un micro-canal ou plusieurs micro-canaux de traitement dont la hauteur intérieure et/ou la largeur intérieure est (sont) comprise(s) dans la gamme de 0,5 mm à 1,5 mm, et dont la longueur est comprise dans la gamme de 5 cm à 100 cm.
